# EUROPÄISCHE PATENTSCHRIFT

(11) **EP 2 929 900 B1**
(45) Veröffentlichungstag und Bekanntmachung des Hinweises auf die Patenterteilung: **20.02.2019**
(21) Anmeldenummer: 15163323.7
(22) Anmeldetag: 13.04.2015
(51) Int. Cl.: A61M 5/20, A61M 5/46, A61M 5/48

(54) **INJEKTIONSVORRICHTUNG ZUM KONTINUIERLICHEN UND GLEICHMÄSSIGEN APPLIZIEREN EINER INJEKTIONSSUBSTANZ**
INJECTION DEVICE FOR CONTINUOUSLY AND UNIFORMLY APPLYING AN INJECTION SUBSTANCE
DISPOSITIF D'INJECTION DESTINÉ À L'APPLICATION CONTINUE ET RÉGULIÈRE D'UNE SUBSTANCE D'INJECTION

(30) Priorität: 11.04.2014 DE 102014005338
(43) Veröffentlichungstag der Anmeldung: 14.10.2015
(73) Patentinhaber: Gerzen, Andreas, 45711 Datteln (DE)
(72) Erfinder: GERZEN, Andreas, 45711 Datteln (DE); KOWITZ, Klaus, 44649 Herne (DE)
(74) Vertreter: Schöneborn, Holger

(56) Entgegenhaltungen:
- FR-A1- 2 895 681
- US-A- 6 132 414
- US-A1- 2006 122 555
- US-A1- 2008 154 188
- US-A1- 2012 065 618

## Beschreibung

Die Erfindung betrifft eine Injektionsvorrichtung zum kontinuierlichen Applizieren einer Injektionssubstanz über eine Applikationslinie. Ein typisches Anwendungsgebiet sind kosmetische und ästhetische Behandlungen. Beispiele hierfür sind die Faltenunterspritzung, Lippen- und Wangenmodellierung oder die Hautauffrischung. Bei der Injektionssubstanz kann es sich insbesondere um Hyaluronsäure handeln.

Bekannte Injektionsvorrichtungen (EP 2 623 146 A1, FR 2 895 681 A1) umfassen eine Spritzenvorrichtung mit einer Kanüle, einen mit der Injektionssubstanz befüllbaren zylinderförmigen Behälter, der an einem Ende einen Auslass aufweist und über den Auslass mit der Kanüle offen verbunden ist und einen Kolben, der im zylinderförmigen Behälter derart angeordnet ist, dass durch die Verschiebung des Kolbens die Injektionssubstanz durch den Auslass und somit durch die Kanüle herauspressbar ist. Dabei dient ein erstes Antriebssystem dazu, den Kolben zu verschieben, während ein zweites Antriebssystem die Spritzenvorrichtung linear entlang der Längsachse verschieben kann. Bei der Applikation verschiebt das erste Antriebssystem den Kolben in Auslassrichtung, um die Injektionssubstanz zu verabreichen. Gleichzeitig verschiebt das zweite Antriebssystem die Spritzenvorrichtung in entgegengesetzter Richtung. Dadurch kann die applizierte Menge der Injektionssubstanz (Injektionsmenge) gleichmäßig über die Applikationslinie verteilt eingebracht werden.

Derartige Injektionsvorrichtungen weisen eine Steuerung auf: Eine Führungsgröße, z. B. die gewünschte Injektionsmenge, wird vor der Applikation in eine Steuerungseinrichtung eingegeben. In Abhängigkeit von der eingegebenen Führungsgröße beeinflusst die Steuerungseinrichtung über Stellglieder (z. B. Antriebssysteme) die Steuerstrecke mit dem Ziel, dass die Steuergröße (z. B. tatsächliche Injektionsmenge) mit der Führungsgröße übereinstimmt. Problematisch bei Steuerungen ist allerdings, dass der Einfluss von Störgrößen in der Steuerstrecke nicht ausgeglichen werden kann. Hierzu fehlt es der Steuerung an einer Rückkopplungseinheit, welche die Steuergröße misst und dem Steuerungssystem zuführt, sowie einem Regler, der die Regeldifferenz (Abweichung zwischen Führungsgröße und Steuergröße) ausgleicht.

Störgrößen können z. B. auf die Eigenschaften der Injektionssubstanzen wie Viskosität oder Kompressibilität zurückzuführen sein. Auch zunehmender Verschleiß an der Injektionsvorrichtung oder Reibung zwischen Kolben und der Innenwand des zylinderförmigen Behälters können sich störend auf die Steuergröße auswirken.

Werden derartige Störgrößen nicht ausgeglichen, kann sich eine ungleichmäßige Verteilung der Injektionssubstanz über die Applikationslinie ergeben, was zu einem unbefriedigenden Behandlungsergebnis führt. Eine korrigierende Nachbehandlung gestaltet sich sehr schwierig, da sich die Injektionssubstanz dann bereits im Gewebe befindet. Einige Injektionssubstanzen bauen sich erst innerhalb langer Zeiträume im Körper ab. Der Patient muss unter Umständen dauerhaft mit dem sichtbaren ästhetischen Mangel leben. In der Regel bezahlen Patienten kosmetische und ästhetische Behandlungen selbst und nehmen dabei mitunter unangenehme Belastungen in Kauf. Auch vor diesem Hintergrund dürfte ein unschönes Behandlungsergebnis für Patienten besonders enttäuschend und frustrierend sein.

Der Erfindung liegt daher die Aufgabe zugrunde, eine Injektionsvorrichtung zum kontinuierlichen und gleichmäßigen Applizieren einer Injektionssubstanz über eine Applikationslinie für kosmetische und ästhetische Behandlungen zu schaffen.

Diese Aufgabe wird mit den im Patentanspruch 1 aufgeführten Merkmalen gelöst.

Demnach weist die Injektionsvorrichtung Mittel zur direkten oder indirekten Erfassung der Menge der Injektionssubstanz (Injektionsmenge) auf, wobei ein Regler in Abhängigkeit von der erfassten Injektionsmenge die Verschiebung des Kolbens und die Verschiebung der Spritzenvorrichtung mittels der Antriebssysteme derart koordiniert, dass dabei die Abgabe der Injektionsmenge gleichmäßig über die Applikationslinie verteilt erfolgt. Dadurch ist es möglich, Abweichungen zwischen der vorgegebenen und der tatsächlichen Injektionsmenge zu erfassen und auszuregeln. Die Injektionsmenge kann dabei direkt, z. B. durch einen Füllstandssensor, oder indirekt, z. B. durch den Verschiebeweg des Kolbens erfasst werden. Bevorzugt ist die Erfassung mittels eines Drehimpulsgebers. Die Koordinierung der Antriebssysteme erfolgt z. B. in der Weise, dass der Vorschub des Kolbens und/oder der Rückzug der Spritzenvorrichtung in Abhängigkeit von der erfassten Injektionsmenge beschleunigt oder verlangsamt wird. Vorzugsweise ist der Regler so beschaffen, dass die Injektionsmenge unabhängig ist von der Stärke und Länge der verwendeten Kanüle. Ggf. weist die Injektionsvorrichtung eine Einstellmöglichkeit für Stärke und/oder Länge der Kanüle auf, da diese Parameter für den aufgebauten Druck von Bedeutung sind.

In einer vorteilhaften Ausführungsform handelt es sich bei den Mitteln zur Erfassung der Menge der Injektionssubstanz um Mittel zur Erfassung des Drucks der Injektionssubstanz im zylindrischen Behälter. Aufgrund des Zusammenhangs zwischen dem Druck der Injektionssubstanz, die sich im zylindrischen Behälter befindet, und der Injektionsmenge, also der Menge Injektionssubstanz, die aus der Kanüle herausgepresst wird, dient der Druck als Indikator für die Injektionsmenge. Wird beispielsweise ein zu hoher Druck gemessen, was auf die Beschaffenheit des Gewebes zurückzuführen sein kann, muss der Vorschub des Kolbens verlangsamt werden. Gleichzeitig sollte auch der Rückzug der Spritzenvorrichtung, d.h. die Bewegung in die Gegenrichtung verlangsamt werden, damit die ausgebrachte Injektionsmenge über die Applikationslinie konstant bleibt.

Vorzugsweise verhindert der Regler bei Überschreitung eines Grenzwertes für den Druck der Injektionssubstanz im zylindrischen Behälter mittels der Antriebssysteme eine weitere Druckerhöhung. Auch eine Druckreduktion ist auf diese Weise möglich. Dadurch wird verhindert, dass es durch Überdruck zu Schäden an der Injektionsvorrichtung und Verletzungen am Patienten kommt. Es ist z. B. schon vorgekommen, dass Kanülen bei automatischen Injektionsvorrichtungen aus ihrer Fassung regelrecht herausgeschossen wurden. Der Patient kann sich aber auch durch eine unter zu hohem Druck verabreichte Injektionssubstanz verletzen, wenn dadurch z. B. Gewebe oder Nerven beschädigt werden.

Die Spritzenvorrichtung selbst ist typischerweise für den einmaligen Gebrauch vorgesehen. Hierbei kann es sich um handelsübliche Spritzen handeln, die in die Injektionsvorrichtung eingesetzt werden. Die Festlegung am dem Auslass abgewandten Ende der Spritzenvorrichtung, an dem sich in der Regel eine umlaufende Schulter befindet, kann mittels einer Überwurfmutter erfolgen. Dabei ist es von Vorteil, als Dämpfung einen zusätzlichen O-Ring zwischen dem Ende der Spritzenvorrichtung und der übrigen Injektionsvorrichtung vorzusehen, um hier einen zu harten Kontakt zwischen Glas- oder Hartkunststoffteilen und Metallelementen zu vermeiden.

Ein zu hoher Druck kann auch dann auftreten, wenn unbeabsichtigt in Gewebe injiziert wird, in welches bereits zuvor eine gewisse Menge der Injektionssubstanz appliziert wurde. Zumeist handelt es sich bei den mit Hilfe der Injektionsvorrichtung applizierten Substanzen um hochviskose Flüssigkeiten, insbesondere um Hyaluronsäure bzw. Hyaluronsäure enthaltendes Wasser, sodass die Injektionsvorrichtung bei erneuter Injektion in ein im Gewebe gebildetes Depot aus bereits zuvor applizierter Injektionssubstanz einen erhöhten Druck aufbaut. Zweckmäßigerweise ist die Injektionsvorrichtung in der Weise eingerichtet, dass der Kolbenvorschub bei Erreichen eines bestimmten Drucks automatisch gestoppt wird und/oder ein Warnsignal an den Anwender abgegeben wird. Das Warnsignal kann insbesondere akustisch oder optisch sein. Anstelle oder zusätzlich zu einer Druckkontrolle kann für den genannten Zweck auch eine Kontrollfunktion für das applizierte Volumen zum Einsatz kommen.

Bei den Mitteln zur direkten oder indirekten Erfassung der Menge der Injektionssubstanz bzw. des Drucks der Injektionssubstanz im zylindrischen Behälter kann es sich um einen Sensor handeln. Alternativ kann beispielsweise der Druck aber auch indirekt gemessen werden, insbesondere über Spannung und/oder Stromstärke, die an einem Elektromotor zum Vorschub des Kolbens anliegen. Sobald der Druck im zylindrischen Behälter ansteigt, hat dies einen Einfluss auf den Elektromotor; insofern kann der Druck und damit der Regulierungsbedarf indirekt ermittelt werden.

Üblicherweise umfasst das erste und/oder das zweite Antriebssystem einen Elektromotor und mechanische Antriebskomponenten, wie etwa ein Zahnradgetriebe und einen Spindelantrieb. Die Drehbewegung der Motorwelle kann dabei mittels des Spindelantriebs in eine lineare Bewegung für die Verschiebung des Kolbens bzw. der Spritzenvorrichtung umgesetzt werden. Der Spindelantrieb besteht in der Regel aus der Gewindespindel und der Spindelmutter. Mittels eines Stirnradgetriebes kann die Drehmomentübertragung vom Motor auf die Gewindespindel bei paralleler Anordnung dieser Bauteile erfolgen. Diese parallele Anordnung schafft, im Gegensatz zu einer Reihenanordnung, eine kompakte Bauweise, da Motor und Gewindestange im Verhältnis zu ihrer Breite sehr lang sind.

Sinnvollerweise ist die Spritzenvorrichtung mit einem entlang ihrer Längsachse mittels des zweiten Antriebssystems verschiebbaren Schlitten befestigt. Auf diese Weise kann eine linear geführte Verschiebung der Spritzenvorrichtung gewährleistet werden. Die Kraftübertragung zwischen dem zweiten Antriebssystem und dem Schlitten erfolgt vorzugsweise über einen Mitnehmer. Dabei kann es sich z. B. um einen Mitnehmerstift handeln, der mit der nicht rotierenden Spindelmutter eines Spindelantriebs verbunden ist. Zugleich ist der Mitnehmerstift formschlüssig mit dem Schlitten verbunden. Die translatorische Bewegung der Spindelmutter wird somit über den Mitnehmerstift auf den Schlitten übertragen.

In einer alternativen Ausführungsform umfasst das erste und/oder das zweite Antriebssystem einen hydraulischen Antrieb. Typische Komponenten eines hydraulischen Antriebs sind Pumpen, Ventile, Leitungen, Pumpenmotor und dergleichen. Hydraulische Antriebe ermöglichen eine kompakte Bauweise. Durch Schlauchleitungen können die Positionen der hydraulischen Antriebskomponenten flexibel und wunschgemäß im Raum angeordnet werden. Motor und Pumpe können beispielsweise fernab der Spritzenvorrichtung positioniert werden.

Bei der Bewegung der Spritzenvorrichtung über das zweite Antriebssystem wird auch die mit dem zylinderförmigen Behälter verbundene Kanüle mitbewegt, sodass das Ziel der Applikation der Injektionssubstanz entlang einer Linie erreicht wird. Die Kanüle kann beliebige Längen aufweisen, vorzugsweise zwischen 12 und 50 mm, bevorzugt zwischen 20 und 30 mm. Im Gegensatz dazu weisen Standardkanülen meist eine Länge von nur 10 bis 15 mm auf. Der Vorteil einer längeren Kanüle liegt darin, dass weniger häufig eingestochen werden muss, da mit einem Applikationsvorgang eine verhältnismäßig große Injektionsmenge verabreicht werden kann. Als nachteilig bei längeren Kanülen erweist sich die höhere Kraft, die vom Anwender bei der Applikation aufgebracht werden muss, da der hohe Kraftaufwand bei manueller Verabreichung mit größerer Ungenauigkeit verbunden ist. Die maschinelle Applikation mit Hilfe der hier beschriebenen Injektionsvorrichtung macht jedoch auch eine Verabreichung unter erhöhtem Kraftaufwand möglich; der Einsatz längerer Kanülen bei maschinell betriebenen Injektionsvorrichtungen wirkt sich somit besonders vorteilhaft in Bezug auf die Anzahl der Applikationsvorgänge, die Behandlungsdauer und das Schmerzempfinden der Patienten aus.

Sicherheitshalber kann um die Kanüle herum eine Auffangvorrichtung angeordnet sein. Entsteht trotz aller Sicherheitsmaßnahmen und Einstellungen an der Injektionsvorrichtung ein Überdruck, der die Kanüle aus ihrer Fassung befördert, kann diese mittels der Auffangvorrichtung sicher aufgefangen werden. Die Auffangvorrichtung kann beispielsweise die Form einer Überwurfmutter aufweisen. Ebenso möglich ist das Vorsehen eines Kanülenhalters, der bewirkt, dass die Kanüle auch bei Entstehen eines Überdrucks nicht abgesprengt wird.

Gemäß einer bevorzugten Ausführungsform weist die Injektionsvorrichtung eine Aspirationsfunktion auf. Bei Aktivierung dieser Funktion wird der Kolben eine bestimmte Strecke vom Auslass des zylindrischen Behälters weg zurückgefahren, um zu prüfen, ob mit der Kanüle ein Blutgefäß getroffen wurde. Sollte dies der Fall sein, kann der Anwender dies am Auftreten einer kleinen Blutmenge erkennen, insbesondere am Übergang zwischen Kanüle und zylindrischem Behälter, da das Blut eingesaugt ("aspiriert") wird. Der Kolben muss hierzu soweit zurückgefahren werden, dass das ggf. angesaugte Volumen ausreichend groß ist, um eingesaugtes Blut erkennen zu können. Da die Kanüle selbst in der Regel metallisch und daher undurchsichtig ist, sollte das Volumen gleich oder größer als das Innenvolumen der Kanüle sein. Sollte Blut zu erkennen, d. h. ein Blutgefäß getroffen worden sein, muss der Anwender die Kanüle neu ansetzen. Die Aspirationsfunktion stellt somit eine Kontrollfunktion zur Verfügung, um die Injektion in eine falsche Körperstelle zu vermeiden, indem sie dem Anwender erlaubt festzustellen, ob unerwünschterweise die Kanüle ein Blutgefäß verletzt hat.

Damit ein Ansaugen möglich ist, sollte gewährleistet sein, dass ein im zylindrischen Behälter vorhandener Stopfen sich mit dem Kolben der Injektionsvorrichtung zusammen zurückbewegt. Dies kann dadurch gewährleistet werden, dass zwischen Kolben und Stopfen eine Verbindung hergestellt wird. Beispielsweise kann der Kolben an seiner dem Stopfen zugewandten Seite über ein kleines Gewinde verfügen, das für einen Formschluss zwischen Stopfen und Kolben sorgt.

Die Aspirationsfunktion ist vorzugsweise so konfiguriert, dass der Kolben nach einer kurzen Zeitspanne wieder um die gleiche Strecke, die er zurückgefahren wurde, vorgeschoben wird, sodass quasi eine Wiederherstellung der Ausgangssituation erfolgt. Die Zeitspanne sollte so bemessen sein, dass dem Anwender erlaubt wird zu erkennen, ob ein Blutgefäß getroffen wurde, gleichzeitig aber keine zu langen Wartezeiten entstehen. Als sinnvoll hat sich eine Zeitspanne von ca. 1 bis 4 s, insbesondere ca. 2 s herausgestellt.

Sinnvoll sind darüber hinaus Bedienmöglichkeiten der Injektionsvorrichtung, um die Spritzenvorrichtung, insbesondere den Schlitten, und/oder den Kolben in eine bestimmte Position zu fahren. Dies kann zum einen hilfreich sein, wenn der mit der Injektionssubstanz befüllte zylindrische Behälter bzw. die Spritzenvorrichtung insgesamt gewechselt werden soll. In diesem Fall fährt typischerweise der Kolben zurück, d.h. aus dem zylindrischen Behälter heraus, und die Spritzenvorrichtung/der Schlitten nach vorne, d.h. in die entgegengesetzte Richtung. Zum anderen kann der Bediener auf diese Weise die Spritzenvorrichtung/den Schlitten je nach Bedarf positionieren.

Zum Vorfahren der Spritzenvorrichtung/des Schlittens kann anstelle eines elektrischen oder hydraulischen Antriebs auch ein elastisches, insbesondere ein Federelement eingesetzt werden. Dieses ist vorzugsweise so ausgebildet, dass es die Spritzenvorrichtung/den Schlitten nach Freigabe durch den Benutzer in eine Position fährt, in der ein Wechsel des zylindrischen Behälters/der Spritzenvorrichtung möglich ist. Sinnvollerweise ist zusätzlich ein Dämpfungselement vorgesehen, um ein zu sprunghaftes Vorfahren zu vermeiden. Das Dämpfungselement kann beispielsweise ein Silikon-Dämpfer sein.

Des Weiteren ist Bestandteil der Erfindung eine Regelung, die dafür sorgt, dass der Kolben beim Stoppen der Applikation jeweils eine kurze Distanz zurückfährt, d.h. vom Auslass des zylindrischen Behälters weg. Auf diese Weise wird der Druck reduziert und der Bildung von unerwünschten Tröpfchen am Kanülenausgang vorgebeugt. Eine derartige Tröpfchenbildung ist unerwünscht, da mit ihr eine unkontrollierte Ausbringung von Injektionssubstanz verbunden ist. Aufgrund des im System aufgebauten Drucks, insbesondere bei hochviskosen Injektionssubstanzen wie Hyaluronsäure, besteht nämlich eine Tendenz des Nachströmens von Injektionssubstanz trotz Stoppen des Kolbenvorschubs. Durch Zurückfahren des Kolbens wird der Druck reduziert und das Nachströmen verhindert. Die Distanz beträgt typischerweise 0,5 bis 3 mm, insbesondere 0,8 bis 2 mm, bevorzugt ca. 1 mm. Die genaue Distanz, die zur Vermeidung der Tröpfchenbildung notwendig ist, hängt von verschiedenen Parametern ab, insbesondere dem Volumen des mit Injektionssubstanz befüllbaren Behälters, das allgemein meist 1 oder 2 ml beträgt, der Länge der für die Injektion verwendeten Kanüle, der applizierten Menge und der Dauer der Applikation. Die Injektionsvorrichtung kann über Einstellmöglichkeiten für die genannten Parameter verfügen, sodass die genaue Distanz automatisch errechnet wird.

Ebenfalls sinnvoll ist ein Modus, in dem der Kolben zur Entlüftung des zylindrischen Behälters an die Injektionssubstanz herangefahren wird, wie es auch bei manueller Handhabung einer Spritze geschieht. Auf diese Weise wird verhindert, dass dem Patienten Luft injiziert wird. Dies ist in erster Linie beim Spritzenwechsel, d. h. beim Wechsel des zylindrischen Behälters von Bedeutung. Die Steuerung bzw. der Regler der Injektionsvorrichtung ist vorteilhafterweise so eingerichtet, dass der Kolben so weit in Auslassrichtung vorgeschoben wird, bis ein leichter Widerstand auftritt. Anschließend kann entweder automatisch ein weiterer, langsamerer Vorschub des Kolbens erfolgen, bis sämtliche Luft aus der Kanüle entfernt ist, wobei die Injektionsvorrichtung ggf. Mittel zur Detektion ausgetriebener Luft oder Flüssigkeit aufweisen kann. Alternativ kann der Anwender manuell den weiteren, langsamen Vorschub des Kolbens steuern, wobei er visuell prüft, ob sich in der Kanüle noch Luft befindet, insbesondere indem er den Kolben so lange vorschiebt, bis eine geringe Menge Injektionssubstanz austritt. Sinnvollerweise ist die Steuerung der Injektionsvorrichtung so eingerichtet, dass anschießend der Kolben eine kurze Distanz zurück, d. h. vom Auslass des zylindrischen Behälters wegfährt, wie es oben bereits beschrieben wurde, um ein unkontrolliertes Austreten von Injektionssubstanz zu vermeiden.

Vorzugsweise kann das zweite Antriebssystem zum Zurückziehen der Spritzenvorrichtung auch deaktiviert werden, beispielsweise wenn der Anwender mit der erfindungsgemäßen Injektionsvorrichtung eine Substanz nicht verteilt über eine Applikationslinie, sondern lokal begrenzt ausbringen möchte oder das Führen entlang der Applikationslinie manuell erfolgen soll.

Das Volumen des mit Injektionssubstanz befüllbaren Behälters kann in üblichen Bandbreiten variieren und liegt typischerweise zwischen 0,5 und 5 ml, zumeist zwischen 1 und 2 ml.

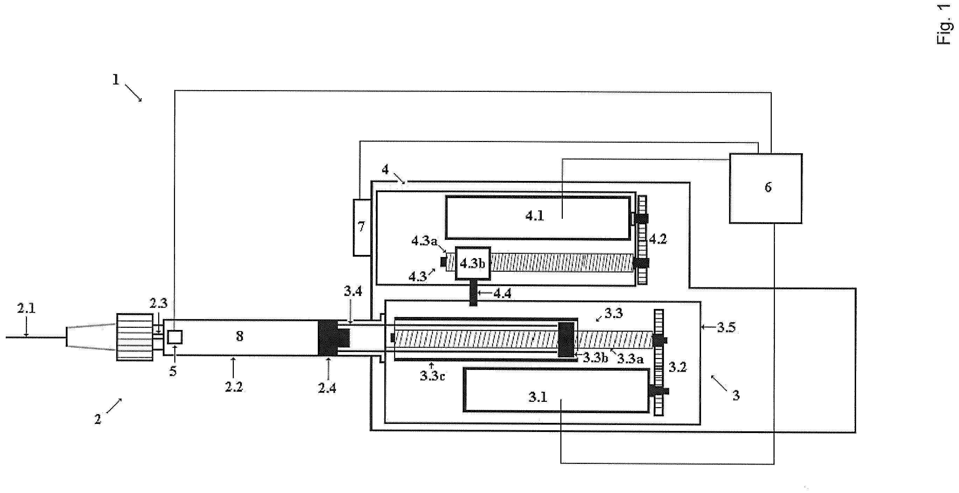

Ein Ausführungsbeispiel der Erfindung wird im Folgenden anhand der Figur 1 näher erläutert:
Figur 1 zeigt die Injektionsvorrichtung 1 mit einer Spritzenvorrichtung 2, einem ersten Antriebssystem 3, einem zweiten Antriebssystem 4, einem Sensor 5 zur Erfassung der Injektionsmenge, einem Regler 6 und einem Bedienfeld 7. Im Ausführungsbeispiel wird zwar die Erfassung der Injektionsmenge über einen Sensor 5 beschrieben, es sei jedoch noch einmal klargestellt, dass ebenso eine indirekte Erfassung möglich ist, beispielsweise über Stromstärke und/oder Spannung des für den Vortrieb des Kolbens 2.4 verantwortlichen ersten Antriebssystems 3.

Die Spritzenvorrichtung 2 umfasst ihrerseits eine Kanüle 2.1, einen zylinderförmigen Behälter 2.2, der an einem Ende einen Auslass 2.3 aufweist, über den er offen mit der Kanüle 2.1 verbunden ist, und einen Kolben 2.4. Die Spritzenvorrichtung 2 ist mit dem ersten Antriebssystem 3 verbunden.

Das erste Antriebssystem 3 umfasst einen Motor 3.1, ein Stirnradgetriebe 3.2 und einen Spindelantrieb 3.3. Der Spindelantrieb 3.3 besteht aus einer rotationsfähigen Gewindespindel 3.3a und einer Vierkantspindelmutter 3.3b, die in einem Vierkantrohr 3.3c angeordnet und dadurch an einer Rotationsbewegung gehindert ist. Über ein Rundrohr 3.4 ist die Vierkantspindelmutter 3.3b mit dem Kolben 2.4 verbunden. Das erste Antriebssystem 3 ist auf einem linear verschiebbaren Schlitten 3.5 befestigt, an dem ebenfalls die Spritzenvorrichtung 2 befestigt ist. Der Schlitten 3.5 ist in Längsachsenrichtung der Spritzenvorrichtung 2 verschiebbar.

Das zweite Antriebssystem 4 besteht aus einem Motor 4.1, einem Stirnradgetriebe 4.2 und einem Spindelantrieb 4.3. Der Spindelantrieb 4.3 umfasst seinerseits eine rotationsfähige Gewindespindel 4.3a und eine nicht rotierende Spindelmutter 4.3b. Die translatorische Bewegung der Spindelmutter 4.3b wird über einen Mitnehmerstift 4.4 auf den Schlitten 3.5 übertragen.

Über ein Bedienfeld 7 oder auch über ein an die Injektionsvorrichtung 1 angeschlossenes, hier nicht dargestelltes Bedienpult kann die gewünschte Injektionsmenge (Sollwert) eingegeben werden. Zusätzlich kann ebenfalls die gewünschte Länge der Applikationslinie (Sollwert) eingegeben werden. Zu berücksichtigen ist darüber hinaus auch die Länge der Kanüle. Aufgrund der eingegebenen Sollwerte ermittelt der Regler 6 die erforderlichen Stellgrößen für die beiden Antriebssysteme, d. h. den Kolbenvorschub und den Schlittenrückzug.

Dabei wird das erste Antriebssystem 3 über den Motor 3.1 angesteuert. Die Drehbewegung des Motors 3.1 bzw. der Motorwelle wird über das Stirnradgetriebe 3.2 auf die Gewindestange 3.3a übertragen. Da die Vierkantspindelmutter 3.3b durch das Vierkantrohr 3.3c an einer Rotationsbewegung gehindert ist, führt sie eine translatorische Bewegung in Kolbenrichtung aus. Dabei verschiebt die Vierkantspindelmutter 3.3b über das Rundrohr 3.4 den Kolben 2.4 in Auslassrichtung. Durch den Kolbenvorschub wird eine Injektionssubstanz 8 in dem zylinderförmigen Behälter 2.2 durch den Auslass 2.3 und aus der Kanüle 2.1 herausgepresst. Der Sensor 5 erfasst dabei die Injektionsmenge, d. h. die Menge der Injektionssubstanz 8, die dem Patienten verabreicht wird, und führt diesen Wert dem Regler 6 zu.

Gleichzeitig wird das zweite Antriebssystem 4 über den Motor 4.1 angesteuert. Die Drehbewegung des Motors 4.1 bzw. der Motorwelle wird über das Stirnradgetriebe 4.2 auf die Gewindestange 4.3a übertragen. Da die Spindelmutter 4.3b nicht rotationsfähig ist, führt diese eine translatorische Bewegung in Gegenrichtung des Kolbenvorschubs aus. Über den Mitnehmerstift 4.4 verschiebt sie dabei den Schlitten 3.5 und somit die Spritzenvorrichtung 2 ebenfalls in Gegenrichtung zum Kolbenvorschub.

Stellt der Regler 6 eine Regelabweichung fest, d. h. eine Differenz zwischen Sollwert (vorgegebene Injektionsmenge) und Istwert (tatsächliche, durch den Sensor erfasste Injektionsmenge), so regelt er diese über die beiden Antriebssysteme aus: Beispielsweise würde der Regler 6 die Drehzahl des Motors 3.1 erhöhen, wenn die tatsächliche Injektionsmenge niedriger ist als die vorgegebene Injektionsmenge. Dadurch würde er die Kolbenvorschubgeschwindigkeit vergrößern. Gleichzeitig oder alternativ könnte er die Drehzahl des Motors 4.1 verringern, um die Injektionsmenge pro Wegeinheit auf der Applikationslinie konstant zu halten.

## Patentansprüche

1. Injektionsvorrichtung zum kontinuierlichen Applizieren einer Injektionssubstanz (8) über eine Applikationslinie, insbesondere für kosmetische und ästhetische Behandlungen mit
- einer Spritzenvorrichtung (2) mit
∘ einem mit der Injektionssubstanz (8) befüllbaren zylinderförmigen Behälter (2.2), der an einem Ende einen Auslass (2.3) aufweist und über den Auslass (2.3) mit einer Kanüle (2.1) offen verbindbar ist,
∘ einem Kolben (2.4), der im zylinderförmigen Behälter (2.2) derart angeordnet ist, dass durch die Verschiebung des Kolbens (2.4) die Injektionssubstanz (8) durch den Auslass (2.3) und die hiermit verbindbare Kanüle (2.1) herauspressbar ist,
- einem ersten Antriebssystem (3) zum Verschieben des Kolbens (2.4),
- einem zweiten Antriebssystem (4) zum linearen Verschieben der Spritzenvorrichtung (2) entlang ihrer Längsachse,
wobei die Applikationslinie entlang der Längsachse der Spritzenvorrichtung (2) verläuft und bei der Applikation der Injektionssubstanz (8) das erste Antriebssystem (3) den Kolben (2.4) in Auslassrichtung verschiebt, während das zweite Antriebssystem (4) die Spritzenvorrichtung (2) in die Gegenrichtung bewegt, um dadurch die applizierte Menge der Injektionssubstanz (8) gleichmäßig über die Applikationslinie verteilt auszubringen, **dadurch gekennzeichnet, dass** die Injektionsvorrichtung (1) Mittel zur direkten oder indirekten Erfassung der Menge der Injektionssubstanz (8) aufweist, wobei ein Regler (6) so eingerichtet ist, dass er in Abhängigkeit von der erfassten Menge der Injektionssubstanz (8) die Verschiebung des Kolbens (2.4) und die Verschiebung der Spritzenvorrichtung (2) mittels der Antriebssysteme (3, 4) derart koordiniert, dass die Menge der Injektionssubstanz (8) über die Applikationslinie konstant bleibt, wobei die Injektionsvorrichtung über eine Regelung verfügt, die bewirkt, dass der Kolben (2.4) beim Stoppen der Applikation eine kurze Distanz entgegen der Auslassrichtung zurückfährt.

2. Injektionsvorrichtung nach Anspruch 1, **dadurch gekennzeichnet, dass** das Mittel zur Erfassung der Menge der Injektionssubstanz (8) ein Mittel zur direkten oder indirekten Erfassung des Drucks der Injektionssubstanz (8) im zylindrischen Behälter (2.2) ist.

3. Injektionsvorrichtung nach Anspruch 2 **dadurch gekennzeichnet, dass** der Regler (6) bei Überschreitung eines Grenzwertes für den Druck der Injektionssubstanz (8) im zylindrischen Behälter (2.2) mittels der Antriebssysteme eine weitere Druckerhöhung verhindert und/oder den Druck reduziert.

4. Injektionsvorrichtung nach einem der Ansprüche 1 bis 3, **dadurch gekennzeichnet, dass** das Mittel zur Erfassung der Menge der Injektionssubstanz (8) ein Sensor (5) ist.

5. Injektionsvorrichtung nach einem der Ansprüche 1 bis 4, **dadurch gekennzeichnet, dass** das erste Antriebssystem (3) einen Elektromotor (3.1) und mechanische Antriebskomponenten, beispielsweise ein Zahnradgetriebe (3.2) und einen Spindelantrieb (3.3), umfasst.

6. Injektionsvorrichtung nach Anspruch 5, **dadurch gekennzeichnet, dass** die Erfassung des Drucks der Injektionssubstanz (8) indirekt über die Messung der Spannung und/oder der Stromstärke am Elektromotor (3.1) erfolgt.

7. Injektionsvorrichtung nach einem der Ansprüche 1 bis 6, **dadurch gekennzeichnet, dass** das zweite Antriebssystem (4) einen Elektromotor (4.1) und mechanische Antriebskomponenten, beispielsweise ein Zahnradgetriebe (4.2) und einen Spindelantrieb (4.3), umfasst.

8. Injektionsvorrichtung nach einem der Ansprüche 1 bis 7, **dadurch gekennzeichnet, dass** das erste oder zweite Antriebssystem (3, 4) einen hydraulischen Antrieb umfasst.

9. Injektionsvorrichtung nach einem der Ansprüche 1 bis 8, **gekennzeichnet durch** ein elastisches Element, insbesondere ein Federelement, welches die Spritzenvorrichtung (2) entlang ihrer Längsachse in eine Position bringt, in der ein Wechsel der Spritzenvorrichtung (2) möglich ist.

10. Injektionsvorrichtung nach einem der Ansprüche 1 bis 9, **dadurch gekennzeichnet, dass** die Spritzenvorrichtung (2) an einem entlang ihrer Längsachse mittels des zweiten Antriebssystems (4) verschiebbaren Schlitten (3.3c) befestigt ist.

11. Injektionsvorrichtung nach einem der Ansprüche 1 bis 10, **gekennzeichnet durch** eine Kanüle (2.1), die eine Länge von 12 bis 50, bevorzugt 20 bis 30 mm aufweist.

12. Injektionsvorrichtung nach einem der Ansprüche 1 bis 11, **dadurch gekennzeichnet, dass** um die Kanüle (2.1) eine Auffangvorrichtung oder an der Kanüle (2.1) ein Kanülenhalter angeordnet ist.

13. Injektionsvorrichtung nach einem der Ansprüche 1 bis 12, **gekennzeichnet durch** eine Aspirationsfunktion, bei deren Aktivierung der Kolben (2.4) soweit entgegen der Auslassrichtung des zylindrischen Behälters (2.2) bewegt wird, dass eine hierbei angesaugte Flüssigkeit in der Spritzenvorrichtung (2) sichtbar wird.

14. Injektionsvorrichtung nach Anspruch 13, **dadurch gekennzeichnet, dass** der Kolben (2.4) nach einer zur Erkennung von angesaugter Flüssigkeit ausreichenden Zeitspanne in Richtung Auslass des zylindrischen Behälters (2.2) bewegt wird.

## Claims

1. Injection device for the continuous application of an injection substance (8) over an application line, in particular for cosmetic and aesthetic treatments, having
- a syringe device (2) with
∘ a cylindrical vessel (2.2) which can be filled with the injection substance (8) and which has an outlet (2.3) at one end and is openly connectable via the outlet (2.3) to a cannula (2.1),
∘ a piston (2.4) which is arranged in the cylindrical vessel (2.2) such that, as a result of the displacement of the piston (2.4), the injection substance (8) can be forced out through the outlet (2.3) and the cannula (2.1) connectable thereto,
- a first drive system (3) for displacing the piston (2.4),
- a second drive system (4) for linearly displacing the syringe device (2) along its longitudinal axis,
wherein the application line runs along the longitudinal axis of the syringe device (2) and, during the application of the injection substance (8), the first drive system (3) displaces the piston (2.4) in the outlet direction, while the second drive system (4) moves the syringe device (2) in the opposite direction in order to thereby dispense the applied quantity of the injection substance (8) in a manner distributed uniformly over the application line, **characterized in that** the injection device (1) has means for directly or indirectly detecting the quantity of the injection substance (8), wherein a controller (6) is configured so as to, in a manner dependent on the detected quantity of the injection substance (8), coordinate the displacement of the piston (2.4) and the displacement of the syringe device (2) by means of the drive systems (3, 4) such that the quantity of the injection substance (8) remains constant over the application line, wherein the injection device has a control regime which has the effect that, upon the stoppage of the application, the piston (2.4) travels backward a short distance counter to the outlet direction.

2. Injection device according to Claim 1, **characterized in that** the means for detecting the quantity of the injection substance (8) is a means for directly or indirectly detecting the pressure of the injection substance (8) in the cylindrical vessel (2.2).

3. Injection device according to Claim 2, **characterized in that**, in the event of a threshold value for the pressure of the injection substance (8) in the cylindrical vessel (2.2) being exceeded, the controller (6) prevents a further pressure increase, and/or reduces the pressure, by means of the drive systems.

4. Injection device according to any of Claims 1 to 3, **characterized in that** the means for detecting the quantity of the injection substance (8) is a sensor (5).

5. Injection device according to any of Claims 1 to 4, **characterized in that** the first drive system (3) comprises an electric motor (3.1) and mechanical drive components, for example a gearwheel mechanism (3.2) and a spindle drive (3.3).

6. Injection device according to Claim 5, **characterized in that** the detection of the pressure of the injection substance (8) is performed indirectly through the measurement of the voltage and/or the current intensity at the electric motor (3.1).

7. Injection device according to any of Claims 1 to 6, **characterized in that** the second drive system (4) comprises an electric motor (4.1) and mechanical drive components, for example a gearwheel mechanism (4.2) and a spindle drive (4.3).

8. Injection device according to any of Claims 1 to 7, **characterized in that** the first or second drive system (3, 4) comprises a hydraulic drive.

9. Injection device according to any of Claims 1 to 8, **characterized by** an elastic element, in particular a spring element, which moves the syringe device (2) along its longitudinal axis into a position in which an exchange of the syringe device (2) is possible.

10. Injection device according to any of Claims 1 to 9, **characterized in that** the syringe device (2) is fastened to a carriage (3.3c) which is displaceable along the longitudinal axis of said syringe device by means of the second drive system (4).

11. Injection device according to any of Claims 1 to 10, **characterized by** a cannula (2.1) which has a length of 12 to 50, preferably 20 to 30 mm.

12. Injection device according to any of Claims 1 to 11, **characterized in that** a collecting device is arranged around the cannula (2.1), or a cannula holder is arranged at the cannula (2.1).

13. Injection device according to any of Claims 1 to 12, **characterized by** an aspiration function, upon the activation of which the piston (2.4) is moved counter to the outlet direction of the cylindrical vessel (2.2) to such an extent that a liquid drawn in in the process becomes visible in the syringe device (2).

14. Injection device according to Claim 13, **characterized in that**, after a time period sufficient for the detection of drawn-in liquid, the piston (2.4) is moved in the direction of the outlet of the cylindrical vessel (2.2).

## Revendications

1. Dispositif d'injection pour l'application en continu d'une substance à injecter (8) sur d'une ligne d'application, plus particulièrement pour des traitements cosmétiques et esthétiques, avec
- un dispositif à seringue (2) avec
• un récipient cylindrique (2.2) pouvant être rempli avec la substance à injecter (8), qui comprend, à une extrémité, une sortie (2.3) et qui peut être relié de manière ouverte par l'intermédiaire de la sortie (2.3), avec une canule (2.1),
• un piston (2.4) qui est disposé dans le récipient cylindrique (2.2) de façon à ce que le coulissement du piston (2.4) permette d'expulser la substance à injecter (8) à travers la sortie (2.3) et à travers la canule (2.1) qui peut être reliée à celle-ci,
- un premier système d'entraînement (3) pour le coulissement du piston (2.4),
- un deuxième système d'entraînement (4) pour le coulissement linéaire du dispositif à seringue (2) le long de son axe longitudinal,
la ligne d'application s'étendant le long de l'axe longitudinal du dispositif à seringue (2) et, lors de l'application de la substance à injecter (8), le premier système d'entraînement (3) faisant coulisser le piston (2.4) en direction de la sortie, tandis que le deuxième système d'entraînement (4) déplace le dispositif à seringue (2) dans la direction opposée afin de répartir de manière uniforme la quantité appliquée de substance à injecter (8) sur de la ligne d'application, **caractérisé en ce que** le dispositif d'injection (1) comprend des moyens pour la mesure directe ou indirecte de la quantité de substance à injecter (8), un régulateur (6) étant conçu pour coordonner, en fonction de la quantité mesurée de substance à injecter (8), le coulissement du piston (2.4) et le coulissement du dispositif à seringue (2) au moyen des systèmes d'entraînement (3, 4), de façon à ce que la quantité de substance à injecter (8) reste constante sur la ligne d'application, le dispositif d'injection disposant d'une régulation qui fait en sorte que le piston (2.4) parcoure à nouveau une courte distance dans la direction opposée à la direction de la sortie lors de l'arrêt de l'application.

2. Dispositif d'injection selon la revendication 1, **caractérisé en ce que** le moyen de mesure de la quantité de substance à injecter (8) est un moyen de mesure directe ou indirecte de la pression de la substance à injecter (8) dans le récipient cylindrique (2.2).

3. Dispositif d'injection selon la revendication 2, **caractérisé en ce que** le régulateur (6) empêche une augmentation supplémentaire de la pression et/ou réduit la pression lors du dépassement d'une valeur limite pour la pression de la substance à injecter (8) dans le récipient cylindrique (2.2), au moyen des systèmes d'entraînement.

4. Dispositif d'injection selon l'une des revendications 1 à 3, **caractérisé en ce que** le moyen de mesure de la quantité de substance à injecter (8) est un capteur (5).

5. Dispositif d'injection selon l'une des revendications 1 à 4, **caractérisé en ce que** le premier système d'entraînement (3) comprend un moteur électrique (3.1) et des composants d'entraînement mécaniques, par exemple un engrenage à roues dentées (3.2) et un entraînement à broche (3.3).

6. Dispositif d'injection selon la revendication 5, **caractérisé en ce que** la mesure de la pression de la substance à injecter (8) a lieu indirectement par l'intermédiaire de la mesure de la tension et/ou du courant au niveau du moteur électrique (3.1).

7. Dispositif d'injection selon l'une des revendications 1 à 6, **caractérisé en ce que** le deuxième système d'entraînement (4) comprend un moteur électrique (4.1) et des composants d'entraînement mécaniques, par exemple un engrenage à roues dentées (4.2) et un entraînement à broche (4.3).

8. Dispositif d'injection selon l'une des revendications 1 à 7, **caractérisé en ce que** le premier ou le deuxième système d'entraînement (3, 4) comprend un entraînement hydraulique.

9. Dispositif d'injection selon l'une des revendications 1 à 8, **caractérisé par** un élément élastique, plus particulièrement un élément à ressort, qui amène le dispositif à seringue (2) de long de son axe longitudinal dans une position dans laquelle un changement de dispositif à seringue (2) est possible.

10. Dispositif d'injection selon l'une des revendications 1 à 9, **caractérisé en ce que** le dispositif à seringue (2) est fixé à un chariot (3.3c) pouvant coulisser le long de son axe longitudinal au moyen du deuxième système d'entraînement (4).

11. Dispositif d'injection selon l'une des revendications 1 à 10, **caractérisé par** une canule (2.1) qui présente une longueur de 12 à 50, de préférence de 20 à 30 mm.

12. Dispositif d'injection selon l'une des revendications 1 à 11, **caractérisé en ce que**, autour de la canule (2.1), est disposé un dispositif de collecte ou sur la canule (2.1), est disposé un support de canule.

13. Dispositif d'injection selon l'une des revendications 1 à 12, **caractérisé par** une fonction d'aspiration qui, lorsqu'elle est activée, permet de déplacer le piston (2.4) dans la direction opposée à la direction de la sortie du récipient cylindrique (2.2) jusqu'à ce qu'un liquide aspiré par celle-ci soit visible dans le dispositif à seringue (2).

14. Dispositif d'injection selon la revendication 13, **caractérisé en ce que** le piston (2.4) est déplacé, après un laps de temps suffisant pour la détection du liquide aspiré, en direction de la sortie du récipient cylindrique (2.2).
